# EUROPEAN PATENT APPLICATION

(11) **EP 1 683 470 A1**
(43) Date of publication of application: **26.07.2006**
(21) Application number: 05112590.4
(22) Date of filing: 21.12.2005
(51) Int. Cl.: A61B 1/005

(54) **Boroscope with Selectable Stiffness Light Shaft**

(30) Priority: 21.12.2004 US 638281 P; 20.12.2005 US 312260
(71) Applicant: CML Innovative Technologies, Inc., Hackensack, NJ 07601 (US)
(72) Inventor: SOSNIAK, Krzysztof, Wallington, NJ 07057 (US); HANES, Robert, Rochelle Park, NJ 07662 (US); REHBERGER, Michael, Kinnelon, NJ 07405 (US); LAGADEC, Gerard, 25021, Cedex (FR)
(74) Representative: Cookson, Barbara Elizabeth

(57) **Abstract**

A boroscope (10) having a flexible fiber optic bundle (30) communicating from inside to outside a housing (12). The bundle (30) has a proximal end inside the housing viewable by viewing optics (26) and illuminable by a light source (16) in the housing. The fiber optic bundle (30) extends to a distal end (52) thereof outside the housing. An external sheath (54) is placeable over the fiber optic bundle and is attachable to a fixture (42) on the housing. The sheath in a preferred form is a tightly coiled wire (55) in goose neck form which is bendable but retains its bent shape for enabling aiming of the distal end of the fiber optic bundle at a remote target.

## Description

### CROSS REFERENCE TO RELATED APPLICATION

This application is based on and claims benefit of U.S. Provisional Patent Application No. 60/638,281, filed December 21, 2004, entitled BOROSCOPE WITH SELECTABLE STIFFNESS LIGHT SHAFT, the disclosure of which is hereby incorporated by reference and to which a claim of priority is hereby made.

### FIELD OF THE INVENTION

The present invention relates to a boroscope and particularly to the light and image fiber optics bundle or shaft attached to the boroscope.

A boroscope, often also referred to as a borescope, transmits an image from a remote target to a viewer's eye, illuminates the target and may magnify the image. There are several types of boroscopes. The invention concerns the fiber optic type of boroscope which employs a flexible channel or shaft that is packed with a bundle of up to tens of thousands of known light transmitting fibers. Each fiber transmits a "pixel" of light from the target being viewed through an eyepiece with the pixels creating a coherent image at the eyepiece. Illumination from a remote source, such as the boroscope itself, passes through light transmitting fibers in the other direction, i.e., toward the point of inspection, to illuminate the target to be viewed. The fiber bundle may extend from the boroscope from ½ meter to 1 meter, or a longer or a shorter distance in a particular application.

Boroscopes have numerous applications in various industries, including without limitation automotive applications which allow inspection for problems of manufacture or subsequent servicing at otherwise hard to see interior areas of engines, transmissions, fuel injectors and other automotive parts, fuel systems including fuel injector nozzles, bodies, pumps and lines, and various engine parts including valves, manifolds, cylinders, sleeves, etc. Boroscopes are useful for looking at aerospace parts during manufacture and during servicing, which parts may be otherwise visually inaccessible. Boroscopes may be used in connection with machining metal, since their fiber bundle shape may be inserted and moved into deep bores, complex castings, bent tubes, etc. and for inspecting rifled barrels. Boroscopes may be used for inspecting interior cavities, holes, etc. The above applications for industry and manufacture are illustrative of a variety of uses. Similar devices used in surgery and medical diagnostics, are known as endoscopes.

### BACKGROUND OF THE INVENTION

The fiber bundle or shaft of a boroscope is typically a non-rigid, somewhat flexible, solid bundle of fibers, having a generally circular cross section, with a diameter dependent upon the number of fibers in the bundle and upon the protective, shape defining sheath around the fiber bundle. In a boroscope embodiment according to the present invention, the light shaft has a diameter of about 5 mm. But, this is an example only.

Boroscopes are generally constructed so that the fiber bundle is relatively flexible to cater to the many uses in which the boroscope shaft must be inserted into a narrow bore or area of the machinery to be observed, and along a path having turns, bends and curves.

In some applications, however, the space into which the fiber bundle shaft is inserted is not narrow, tight and confined. The distal illumination and viewing tip of the shaft is then not easily directed at the target to be viewed, because it will sag or move off the desired aiming direction. In order to deal with that, boroscopes are available which include a stiffened fiber bundle or shaft or one that is capable of being fixedly oriented as a particular application requires. This increases the weight since the stiffened light shaft is usually thicker and heavier, but it does permit the shaft to maintain its selected orientation.

Such a sheath permanently disposed around the fiber bundle might be a round reinforcing wire, or the like, coiled tightly into what is sometimes referred to as a "goose neck". Such a coiled wire sheath can be given any selected curvature along its length and the entrance and exit of light from the distal free end or tip thereof can be in a controllable direction and toward a target. Other types of stiffening sheaths may also be used.

Boroscopes are relatively expensive instruments, primarily as a result of their fiber optic bundles, but also from the internal elements of the boroscope which illuminate the fiber bundle and from the optics that receive and focus the target image collected. Accordingly, it would be desirable to maximize uses for a boroscope by providing a fiber bundle shaft of small diameter which can also be precisely aimed at a target, depending upon a particular use for the boroscope at the time.

Up to now, however, it has been necessary to employ separate boroscopes for these two types of uses.

### SUMMARY OF THE INVENTION

It is an object of the present invention to enable a boroscope to selectively either have a smaller cross-section more flexible light shaft or a more rigid fiber bundle shaft which more precisely aimable, as the user selects at the time of use.

According to the invention, a conventional boroscope with standard flexible light fiber bundle illumination and target viewing fiber filaments and with a standard boroscope viewing element is provided with a detachable outer sheath which surrounds the length of the fiber bundle sheath to provide rigidity. When the sheath is removed, the fiber bundle shaft exhibits the necessary flexibility to pass through narrow curved passages.

At the option of the user of the boroscope, a hollow, tubular light external sheath is installed over the flexible light shaft or fiber bundle. The tubular external sheath has an internal diameter that is minimally greater than the external diameter of the light fiber bundle. The body of the boroscope and the external sheath have cooperating attachment fixtures where the external sheath meets the boroscope body for enabling attachment of the external sheath to the boroscope body after the sheath has been installed over the light shaft or fiber bundle and also for enabling detachment of the external sheath from the body when it is desired to remove the stiffening external sheath.

In one exemplary, and non-limiting embodiment, a short-length threaded shank is provided in the proximal region of the light fiber bundle at the exit of the fiber bundle from the body of the boroscope. The shank cooperates with complementary attachment fixture at the proximal end of the external sheath. A threaded connection may be provided between the sheath fixture and the body fixture to permit attachment and detachment of the external sheath to the boroscope. The external sheath is about of the length of a fiber bundle so as not to interfere with projection of light toward the target from the distal tip of the fiber bundle or with reception of an image from the target by the distal tip of the fiber bundle.

As will be appreciated by those skilled in the art, other means for coupling the external sheath to the boroscope may be employed.

The external sheath allows the light shaft with its own sheath to be bent along the length and shaped so that the light exiting the fiber optic bundle and the target image received there may be more precisely targeted. The external sheath is of a type which can be curved and configured as a particular application may require and which will retain that shape for the fiber bundle or light shaft. Again, by way of a non-limiting example, the external sheath may be comprised of a coiled stiff wire, optionally encased in a plastic covering, which holds successive windings of the wire in close contact, a design commonly referred to as a "goose neck". Other stiffening arrangements of the external sheath, with memory for whatever shape they are temporarily given, may instead be used.

Other objects and features of the present invention will become apparent from the following description of one preferred embodiment considered in conjunction with the accompanying drawings in which:

### BRIEF DESCRIPTION OF THE DRAWINGS

Fig. 1 is a partially cross-sectioned elevational view of a boroscope with an additional light fiber bundle shaping external sheath according to the invention;

Fig. 2 is a view of the boroscope without the sheath;

Fig. 3 is a view of the boroscope with the fiber bundle and the separate external sheath;

Fig. 4 is a view of the boroscope with the external sheath assembled on the fiber bundle;

Fig. 5 is a fragmentary view of the fiber bundle at A in Fig. 4 and in cross section.

Fig. 6 is a transverse cross-section on 6-6 in Fig. 5;

Fig. 7 is an enlarged cross-section of the exit region for the fiber bundle at the boroscope;

Fig. 8 is a fiber bundle end view of the boroscope.

### DESCRIPTION OF PREFERRED EMBODIMENTS

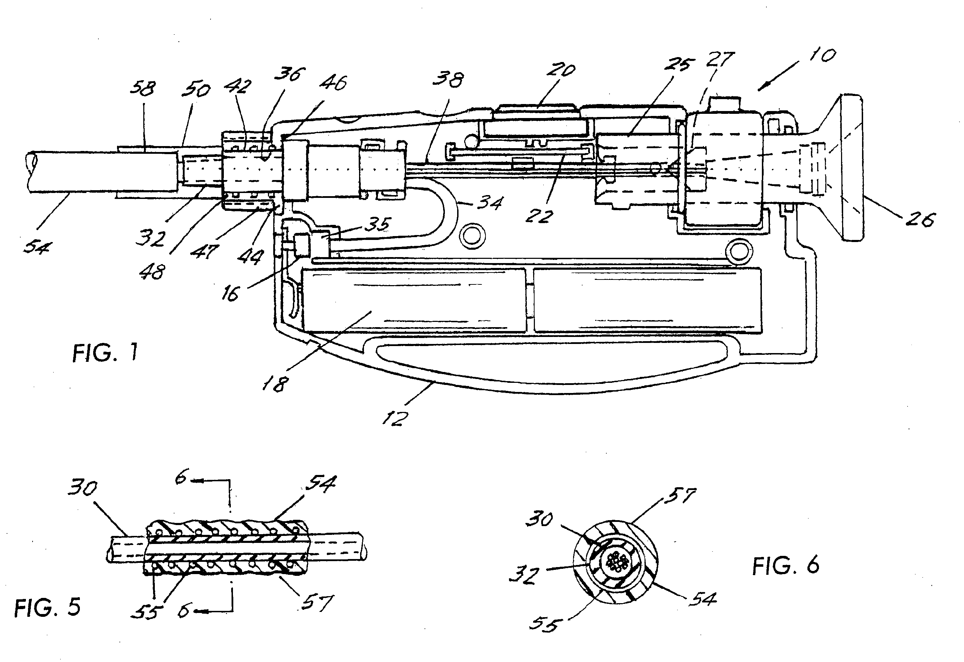

A boroscope 10 to which the present invention is applied is shown in Fig. 1. Its major components include an external housing 12, and elongated fiber-bundle shaft 30, a light source 16 disposed in the housing 12 for illuminating a target object, and an imaging system 25 also disposed in housing 12, including a viewer 26.

Light source 16 is preferably a light emitting diode, but may be an incandescent light source, or a source of light in any wavelength range including invisible, infrared and ultraviolet.

An electric power supply 18 is supported in the housing and is connected by leads or in any other suitable or desired manner to light source 16. In the illustrated embodiment, the supply is an electric battery, and such as two AA size batteries. These may be conventional disposable batteries. Alternatively, a conventional rechargeable battery pack may be provided, or individual rechargeable batteries may be used.. A push button switch 20 may be used in conjunction with a contacts board 22 to complete a circuit from the power supply 18 to the light source 16. Other power switch arrangements may also be provided.

Imaging system 25 is designed to receive an image of the object under inspection returned by the light fibers in fiber-bundle shaft 30. Imaging system 25 may be a conventional optical system comprised of one or more lenses, generally indicated at 27, to focus an image of the target for viewing through an eye piece 26 which opens outwardly at the rear of the boroscope. Alternatively, an electronic imaging system of any conventional or suitable type may be provided, including an image sensor such as a charge coupled device, signal processing devices for amplification, image enhancement, etc. and light emitting diode display. Image recording capability can also be provided, if desired. Suitable means for implementation of such features will be apparent to those skilled in the art.

The body of the boroscope 10 is shaped and sized to be comfortably held in one hand with the eyepiece 26 at the eye and the fiber bundle projecting outwardly of the body to be directed toward the target area.

As is conventional, the light fiber bundle 30 is comprised of many, perhaps as many as several hundred or even several thousand, fine glass filaments or fibers bundled together with a thin sheath 32 around and defining the bundle. The light fibers transmit "pixels" which together define an image when viewed after being focused.

The fiber bundle actually includes two sets of fibers. One set 34 provides illumination of the target object, and is arranged with its proximal end 35 optically coupled to the light source 16. With light source 16 positioned and oriented as illustrated in Fig. 1, the proximal end 35 of fiber set 34 is bent toward bundle exit point 36 from the housing, but as will be obvious to those skilled in the art, other configurations may be employed as required by the placement and orientation of the light source.

A second set of fibers 38 transmits the image of the target object to optical system 25. With the orientation of bundle exit point 36 and the configuration of the internal parts of boroscope 10 as illustrated, image fiber-set 38 extends straight to the focusing optics 25. Again, other configurations may be employed, as necessary or desired.

The two fiber sets 34, 38 are bundled together in the bundle 30 in a conventional manner. The combined light fiber bundle 30 extends from inside the housing 12 and projects through the exit point 36 to outside the housing. The bundle 30 and a surrounding protective sheath 32 are firmly held at exit point 36 in a support fixture 42 which is secured in the housing wall in any conventional or desired manner.

One exemplary, but non-limiting construction for fixture 42 includes a shaped portion 44 on its interior end which engages with the interior 46 of the housing. A locking piece 47, e.g., a lock nut positioned around the exterior of the fixture 42 just outward of the housing wall is tightened over a projecting part 48 of the fixture 42 and with the part 44 of the fixture 42 engaged on the inside of the housing wall, the nut cooperates to securely hold the fixture 42 and therefore the light fiber bundle 30 securely at the housing wall.

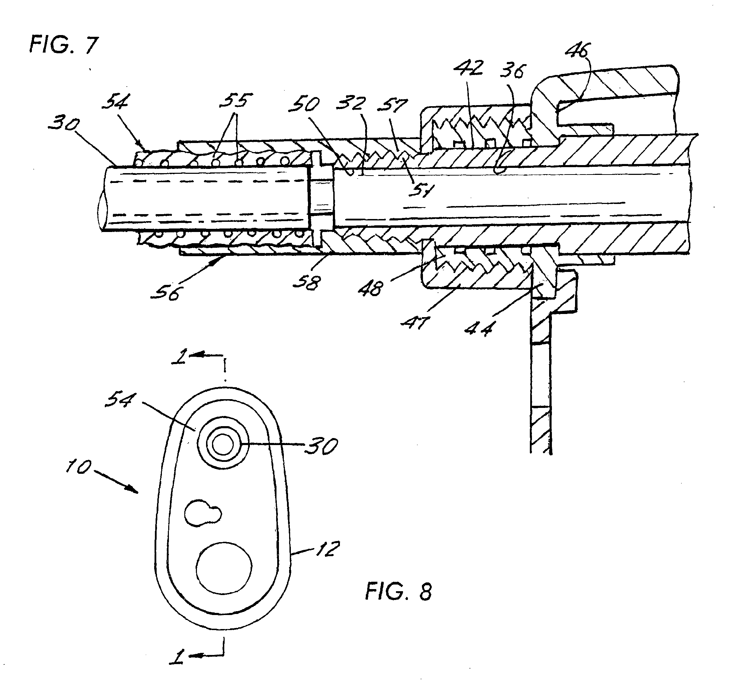

Projecting outwardly from light fiber bundle securement fixture 42 is a sleeve portion 50 best illustrated in Figs. 1 and 7. This may be integral with fixture 42, or attached in any suitable manner. Sleeve 50 is externally threaded at 51 (see Fig. 7) to receive an attaching fixture 56 for an external shape retention and aiming sheath 54 as described below. Light fiber bundle 30 extends from inside the housing 12 through sleeve 50 and then out to a distal tip 52 defining the end of the fiber optic bundle. The distance to tip 52 is dictated by the application, and may, for example, be in the range of from one half meter to one meter, but longer or shorter lengths are obviously possible.

Light from the light source 16 within the boroscope passing through fiber set 34 exits through the tip 52 to illuminate the target. Similarly, an image of the illuminated target enters fiber set 38 through tip 52 and passes to optical system 25.

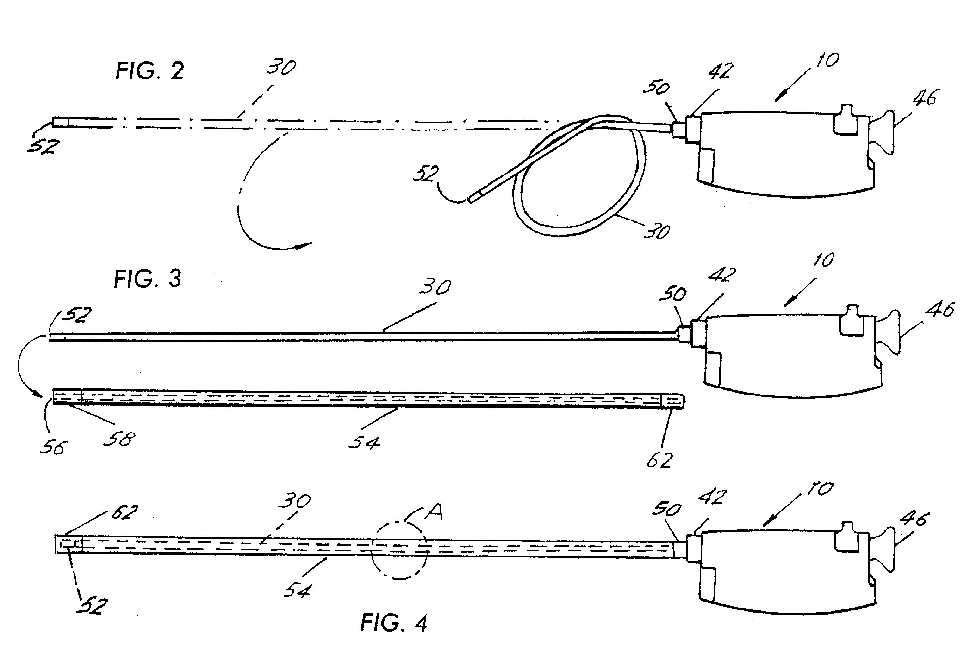

The light fiber bundle 30 is constructed to be sufficiently flexible and bendable to pass through a narrow and curving path to the target object, as shown in Fig. 2. In that condition, however, it is not sufficiently rigid to hold a shape, or to be accurately aimable in a relatively open passage to permit precise illumination and viewing of desired portions of the target object.

To overcome this problem, which still permitting use of the boroscope for applications requiring a flexible, small-diameter fiber-bundle shaft 30, a separate external shape retaining and aiming sheath 54 as seen in Fig. 3 is placed over the protective sheath 32 of the flexible light fiber bundle 30 as seen in Fig. 4, and is detachably retained thereon, as described below. Sheath 54 may be comprised of a tightly wound, steel wire coil 55, covered with a thin protective plastic layer 57. The coils preferably so tightly wound and held together that the external sheath can be bent in any desired manner along its length and will retain whatever shape it is given. Such a construction is often referred to by the term "goose neck". As will be understood by those skilled in the art, however, any other construction for sheath 54 which functions similarly may be employed, if desired.

With the construction according to the invention, when boroscope 10 is used to inspect a target in a relatively open passage, sheath 54 holds a set shape. Thus, if the light emitting and target viewing tip 52 of bundle 30 is placed at or aimed at the target, the tip can be precisely oriented and positioned.

As seen in Figs. 1 and 7, in this preferred exemplary embodiment, the wire 55 of the external sheath 54 is wound to define a hollow tubular shape with an internal diameter only slightly greater than the external diameter of the sheath 32 on the light fiber bundle 30 so that the external sheath 54 may be slid onto the inner sheath 32, which is thereby received in the sheath 54.

To detachably secure external sheath 54 to the boroscope body 12, the proximal end 56 of the external sheath 54 is provided with a fixture 58 for engaging with the cooperating sleeve 50. Fixture 58 is rotatably captured or permanently secured on external sheath 54 in any other suitable manner, and is internally threaded at 57 to engage with external threads 51 on sleeve 50. As shown in Fig. 4, the length of the external sheath 54 is selected so that when it is secured to sleeve 50, its open distal end 62 does not interfere with illumination of the target or the viewing of the target.

The construction described enables a user to employ the same boroscope instrument to view a target which requires the boroscope light fiber bundle to pass through a narrow closely confined curved path which may preclude use of a stiffened external covering sheath or selectively enables a user to apply the covering sheath so that the light emitting and image receiving tip of the boroscope is aimed at the target without the light fiber bundle being difficult to aim or bending or drooping off the target.

Although the present invention has been described in relation to a particular embodiment thereof, many other variations and modifications and other uses will become apparent to those skilled in the art. It is intended, therefore, that the present invention not be limited by the specific disclosure herein, but that it be given the full scope permitted by the appended claims.

## Claims

1. A boroscope comprising:
a housing (12);
a light source (16) and an imaging system (25, 26)) located in the housing (12) ;
a fiber optic bundle (30) extending out of the housing, the fiber optic bundle being comprised of:
a first portion comprised of a first set of fibers (34) coupled at a proximal end to the light source (16) for transmitting light to a distal end thereof to illuminate a target; and
a second portion comprised of a second set of fibers (38) adapted to receive light from a target at a distal end (52) thereof, and to pass the received light to a proximal end thereof for coupling to the imaging system (25, 26)) to enable viewing of the target,
the fiber bundle (30) having such flexibility along its length as to be able to follow the curvature of a passage having a cross sectional dimension not substantially exceeding a cross sectional dimension of the fiber bundle, but not able to retain a set shape in a larger passage having a cross sectional dimension substantially exceeding that of the fiber bundle, thereby hindering precise targeting of the distal end of the bundle in such a larger passage;
an external sheath (54) removably installable over the fiber bundle (30), and releasably securable at a proximal end thereof when so installed,
the external sheath (54) being so comprised that its shape is self-sustaining when
bent or curved along its length, is of such length that the distal end of the fiber bundle may be aimed and positioned at the target to provide illumination thereof, and to receive an image thereof for transmission to the imaging system.

2. The boroscope of claim 1, wherein the external sheath (54) is comprised of a wound coil (55) of a material with sufficient stiffness that the set bent or curved shape is self sustaining.

3. The boroscope of claim 2, wherein the external sheath is a tightly wound coil (55) of wire in a goose neck style.

4. The boroscope of any one of the preceding claims, wherein the external sheath is of such length that when it is secured in its operative position, a distal end (62) of the external sheath (54) is about at the distal end (52) of the fiber bundle (30).

5. The boroscope of any one of the preceding claims, wherein the housing (12) is shaped to be held in the hand and the imaging system (25, 26) includes a viewer (26) on one side of the housing.

6. The boroscope of claim 5, wherein the viewer (25) and a first fixture (42) are at opposite sides of the housing from each other.

7. The boroscope of any one of the preceding claims, further including a first fixture (42) on the exterior of the housing (12) through which the light fiber bundle (30) exits the housing and which secures the bundle to the housing; and a second fixture (58) at a proximal end (56) of the external sheath (54) which is releasably securable to the first fixture (42).

8. The boroscope of claim 7, wherein the second fixture (58) is releasably securable to the first fixture (42) by a threaded connection (51, 50).

9. The boroscope of claim 7, wherein the external sheath (54) is releasably securable by a threaded connection to a sleeve (50) extending from the first fixture (42).

10. The boroscope of claim 1, wherein the external sheath (54) is releasably securable by a threaded connection.

11. The boroscope of claim 1, further including a first fixture (42) on the exterior of the housing (12) through which the light fiber bundle (30) exits the housing and which secures the bundle to the housing; and wherein the external sheath (54) is releasably securable to the first fixture (42).

12. The boroscope of any one of the preceding claims, wherein the external sheath (54) is a tightly wound coil of wire encased in a plastic material.

13. The boroscope of claim 12, wherein the wire is formed of steel.

14. The boroscope of any one of the preceding claims, wherein the imaging system (25) includes at least one lens which focuses an image of the target on an eyepiece.

15. The boroscope of any one of the preceding claims, wherein the imaging system (25) includes an electronic image processing unit and an electronic display.

16. The boroscope of claim 15, wherein the imaging system (25) further includes an image recording unit.
